(19) Europäisches Patentamt
European Patent Office
Office européen des brevets

(11) **EP 4 081 338 B1**

(12) **EUROPEAN PATENT SPECIFICATION**

(45) Date of publication and mention
of the grant of the patent:
**07.02.2024 Bulletin 2024/06**

(21) Application number: **20797505.3**

(22) Date of filing: **03.11.2020**

(51) International Patent Classification (IPC):
***B01J 13/14*** *(2006.01)*     ***A61K 8/11*** *(2006.01)*
***C09B 67/02*** *(2006.01)*     ***C11D 3/50*** *(2006.01)*

(52) Cooperative Patent Classification (CPC):
**A61K 8/25; A61K 8/11; A61Q 5/02; B01J 13/14;**
**C09B 67/0097;** A61K 2800/412; A61K 2800/56

(86) International application number:
**PCT/EP2020/080728**

(87) International publication number:
**WO 2021/129968 (01.07.2021 Gazette 2021/26)**

(54) **MICROCAPSULES AND COSMETIC COMPOSITIONS COMPRISING THE SAME**

MIKROKAPSELN UND KOSMETISCHE ZUSAMMENSETZUNGEN DAMIT

MICROCAPSULES ET COMPOSITIONS COSMÉTIQUES LES COMPRENANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.12.2019 PCT/CN2019/128230**
**15.01.2020 EP 20151923**

(43) Date of publication of application:
**02.11.2022 Bulletin 2022/44**

(73) Proprietors:
• **Unilever IP Holdings B.V.**
**3013 AL Rotterdam (NL)**
Designated Contracting States:
**AL AT BE BG CH CZ DK EE ES FI FR GR HR HU
IS LI LT LU LV MC MK NL NO PL PT RO SE SI SK
SM**
• **Unilever Global IP Limited**
**Wirral, Merseyside CH62 4ZD (GB)**
Designated Contracting States:
**CY DE GB IE IT MT RS TR**

(72) Inventors:
• **PAN, Xiaoyun**
**Shanghai, 200335 (CN)**
• **ZHOU, Weizheng**
**Shanghai, 200434 (CN)**
• **TANG, Yi**
**Shanghai, 200433 (CN)**
• **LEI, Siqi**
**Shanghai, 200433 (CN)**

(74) Representative: **Chisem, Janet**
**Unilever Patent Group**
**Bronland 14**
**6708 WH Wageningen (NL)**

(56) References cited:
WO-A1-2010/070987    WO-A1-2013/174921
WO-A1-2015/091705    WO-A1-2015/091877
WO-A1-2018/054719    WO-A2-2009/063257
US-A1- 2016 177 156    US-A1- 2016 346 218

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the Invention

The present invention relates to microcapsules as well as a cosmetic composition comprising microcapsules.

### Background of the Invention

Many personal care and home care products seek to deliver benefit agents to substrates such as hair, skin, textiles and hard surfaces. To achieve a long-lasting performance, encapsulation of the benefit agent in microcapsule has been proposed as a means, in particular for the perfume. When applied, the microcapsules may be deposited onto the substrates, for example onto skin, and broken by action of pressure and/or rubbing. The perfume is released and brings superior sensory to the consumers.

However, there is still much room for improvement as consumer preference for improved fragrance perception at multiple stages of the wash process, such as at the end of the wash, and forever longer lasting perfume performance, drives commercial interest in this area.

Leakage of encapsulates remains a problem which not only causes reduced performance but also restricts the materials that can be used as protective wall material for encapsulates. Melamine formaldehyde capsules are well-known, but, disadvantageously, need to be used in conjunction with a formaldehyde scavenger.

There is an increasing interest to develop environmental-friendly microcapsules with safety to develop compositions with such microcapsules particles that yield desired performance.

US 2016/177156 A1 discloses thermally conducting capsules for increasing the thermal conductivity and the heat capacity of heat-exchanging materials or also of thermal fluids. It does not disclose the benefit agents which can be used in the cosmetic composition and has nothing to do with the loading/releasing of the benefit agents.

### Summary of the Invention

We developed microcapsule comprising benefit agent in the core and having a shell comprising silica wherein said shell comprises plate-like inorganic materials having an average thickness of 1-1000nm. It was surprisingly found that the such microcapsules could have good loading efficiency and long-lasting release for benefit agent while maintain acceptable leakage for storage when they were included into cosmetic composition.

In accordance with a first aspect of the invention is disclosed a microcapsule comprising

(i) a core comprising a benefit agent; and
(ii) a shell comprising silica; wherein said shell comprises plate-like inorganic materials having an average thickness of 1-1000nm; wherein said benefit agent is fragrance, pro-fragrance, hair conditioning agent, anti-dandruff agent, moisturizers, emollients, dyes and/or pigments, colour care additives or a mixture thereof; wherein the core of said microcapsule comprises a thickening agent.

In accordance with a second aspect of the invention is disclosed a process to prepare a microcapsule of the first aspect of this invention, comprising the steps of:

a) Preparing an oil phase liquid comprising a silica precursor and a benefit agent;
b) Preparing an aqueous suspension by homogenizing the mixture of plate-like inorganic material and deionized water.
c) Preparing an o/w emulsion by adding the aqueous suspension of plate-like inorganic material into the oil phase liquid and homogenized to form emulsion.
d) Putting the o/w emulsion into 40°C oven for at least 24 h to get the microcapsule slurry product; wherein oil phase liquid of step a) comprises a thickening agent.

In accordance with a third aspect is disclosed a cosmetic composition comprising a microcapsule of the first aspect.

### Detailed Description of the Invention

For the avoidance of doubt, any feature of one aspect of the present invention may be utilized in any other aspect of the invention. The word "comprising" is intended to mean "including" but not necessarily "consisting of or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated. As used herein, the indefinite article "a" or "an" and its corresponding definite article "the" means at least one, or one or more, unless specified otherwise. The various features of the present invention referred to in individual sections above apply, as appropriate, to other sections *mutatis mutandis.* Consequently, features specified in one section may be combined with features specified in other sections as appropriate. Any section headings are added for convenience only and are not intended to limit the disclosure in any way.

By "a cosmetic composition" as used herein, is meant to include a composition for topical application to the skin of mammals, especially human beings. Such a composition may be generally classified as leave-on or rinse off but is preferably of the leave on type. The composition is formulated into a product which is applied to a human body specifically for improving appearance but may, in addition, also provide cleansing, odor control or general aesthetics. The composition of the present invention can be in the form of a liquid, lotion, cream, foam, scrub, gel, or toner, or applied with an implement or via a face mask or a pad. Non-limiting examples of such compositions include leave-on skin lotions, creams, antiperspirants, deodorants, lipsticks, foundations, mascara, sunless tanners and sunscreen lotions. The composition of the present invention is preferably a leave-on composition. "Skin" as used herein is meant to include skin on the face and body (e.g., neck, chest, back, arms, underarms, hands, legs, buttocks and scalp) and especially to the sun exposed parts thereof.

By "hair care composition" as used herein, is meant to include a composition for topical application to hair or scalp of mammals, especially humans. By topical is meant that the product is applied to the external surface of the body. In the present invention this is achieved by applying the hair care composition to hair or scalp. Such a product may be generally classified as leave-on or rinse off, and includes any product applied for improving the appearance, cleansing, odor control or general aesthetics of scalp and hair. The hair care composition of the present invention is preferably a leave-on product. Alternatively, the hair care composition of the present invention is a wash-off composition. A hair care composition in accordance with the present invention is preferably a shampoo, hair conditioner, hair cream, hair serum, mousse, hair gel or hair oil.

"Length", "width" and "thickness" refers to the length, width and thickness of particles or microcapsule in an unaggregated state. The term "length" refers to the average dimension of a particle or microcapsule typically along the longitudinal axis. The term "width" refers to the average dimension of the particle or microcapsule perpendicular to the length and typically perpendicular to the longitudinal axis. The terms "thickness" refer to the average dimension of the particle or microcapsule that is perpendicular to the length and width. The length, width and thickness may be measured for example by scanning electron microscopy (SEM) by averaging the values of at least ten particles. "Particle size" as used herein refers to particle diameter in non-aggregated state unless otherwise stated. For polydisperse samples having particulate with diameter no greater than 1 micron, diameter means the z-average particle size measured, for example, using dynamic light scattering (see international standard ISO 13321) with an instrument such as a Zetasizer Nano™ (Malvern Instruments Ltd, UK). For polydisperse samples having particulate with diameter greater than 1 micron, diameter means the apparent volume median diameter (D50, also known as x50 or sometimes d(0.5)) of the particles measurable for example, by laser diffraction using a system (such as a Mastersizer™ 2000 available from Malvern Instruments Ltd) meeting the requirements set out in ISO 13320.

In accordance with a first aspect is disclosed a microcapsule comprising:

(i) a core comprising a benefit agent; and
(ii) a shell comprising silica; wherein said shell comprises plate-like inorganic materials having an average thickness of 1-1000nm; wherein said benefit agent is fragrance, pro-fragrance, hair conditioning agent, anti-dandruff agent, moisturizers, emollients, dyes and/or pigments, colour care additives (including dye fixing agents), or a mixture thereof; wherein the core of said microcapsule comprises a thickening agent.

The term microcapsule means core-shell microcapsule preferably ranging in size from 0.5 to 100 microns, more preferably from 2 to 50 microns, furthermore preferably from 5 to 30 microns, most preferably from 5 to 20 microns.

It is preferred that the thickness of the shell is from 0.01-10 microns, preferably 0.05-1 microns, more preferably 0.1-0.3 microns.

Preferably the microcapsule of the invention is powdery or in the form of an aqueous suspension, more preferably in the form of an aqueous suspension. Such an aqueous form of the microcapsule preferably contains from 1 to 90 wt% microcapsule and the balance being water. Alternatively, the microcapsule is powdery, preferably a freeze-dried powder.

The shell of the microcapsule according to the present invention comprises silica. Preferably, the silica is well dispersed in the shell and combines with the plate-like inorganic materials.

The core of the microcapsule according to the present invention comprises a thickening agent. The thickening agent can be a lipid which is solid at room temperature but is liquified via heat, or a mineral which can absorb oils and boost viscosity. Preferably, the thickening agent can be a lipid selected from fatty alcohol, fatty acid and wax. Preferably, the thickening agent can be a mineral selected from silica, bentonite and magnesium aluminum silicate. More preferably the thickening agent is hydrophobic silica.

Plate-like inorganic materials

The shell of the microcapsule according to the present invention also comprises plate-like inorganic materials.

The inorganic materials of the invention are plate-like and have an average thickness of 1-1000nm, preferably 5-500nm, more preferably 10-100nm, furthermore preferably 10 to 50nm.

It is preferred that the average particle size of the inorganic material is from 10 to 2000nm in diameter, more preferably 50-1000nm, most preferably 100-500nm.

It is preferred that inorganic material is selected from MgAl-layered double hydroxide, hydroxyapatite, diatomite, magnesium hydroxide, calcium hydroxide, zeolite MCM-22, boron nitride or a combination thereof, more preferably the inorganic material is MgAl-layered double hydroxide.

It is preferred that the inorganic material is anionic surface modified. It is preferred that anionic surface modified inorganic material comprises groups selected from sulphate, hydrosulfite, hyposulphite, sulphite, bisulphate, bisulfite, carbonate, bicarbonate, hydroxyl, chlorate, perchlorate, chlorite, hypochlorite, chromate, chromite, bichromate, iodate, nitrate, nitrite, phosphate, hypophosphite, superphosphate, phosphite, monohydrogen phosphate, dihydrogen phosphate, manganate, permanganate, thiosulfate, hydrosulfate, silicate, metasilicate, aluminosilicate, acetate, formate, oxalate, dioxalate, hydrogen sulfide, cyanate, thiocyanate, bomide, chloride, fluoride, iodide, borate, bromate, hypobromite. It is more preferred that the anionic surface modified inorganic material comprises groups selected from sulphate, hydrosulfite, hyposulphite, sulphite, bisulphate, bisulfite, carbonate, bicarbonate, hydroxyl, chlorate, perchlorate, chlorite, hypochlorite, chromate, chromite, bichromate, phosphate, hypophosphite, superphosphate, phosphite, monohydrogen phosphate, dihydrogen phosphate, thiosulfate, hydrosulfate, silicate, metasilicate, aluminosilicate, acetate, formate, oxalate, dioxalate, hydrogen sulfide. It is furthermore preferred that the anionic surface modified inorganic material comprises sulphate group.

It is most preferred that the inorganic materials comprise anionic surface modified MgAl-layered double hydroxide.

It is preferred that the inorganic materials have multilayers and the interlayer distance is from 0.1 to 10nm, more preferably from 0.2 to 5nm, furthermore preferably from 0.5 to 2nm. The inorganic materials preferably have a specific surface area of 5 to 1000 $m^2/g$, more preferably 10 to 500 $m^2/g$, furthermore preferably 50 to 200 $m^2/g$.

Benefit agents

Benefit agents according to the present invention may refer to agents which may provide a range of benefits to hair and/or scalp, and more preferably to human hair. The benefit agent is typically present in an amount of from 10 to 90% by total weight of the microcapsule, more preferably from 30 to 80% by total weight of the microcapsule.

The benefit agents of the present invention include fragrance, pro-fragrance, hair conditioning agent, anti-dandruff agent, moisturizers, emollients, dyes and/or pigments, colour care additives (including dye fixing agents), or a mixture thereof. More preferably, the benefit agent comprises fragrance, pro-fragrance, hair conditioning agent, anti-dandruff agent or a mixture thereof. Furthermore preferably, the benefit agent is selected from a fragrance, pro-fragrance or a mixture thereof and most preferably the benefit agent is a fragrance.

Useful components of the fragrance include materials of both natural and synthetic origin. They include single compounds and mixtures. Specific examples of such components may be found in the current literature, e.g., in Fenaroli's Handbook of Flavour Ingredients, 1975, CRC Press; Synthetic Food Adjuncts, 1947 by M. B. Jacobs, edited by Van Nostrand; or Fragrance and Flavour Chemicals by S. Arctander 1969, Montclair, N.J. (USA). These substances are well known to the person skilled in the art of perfuming, flavouring, and/or aromatizing consumer products, i.e., of imparting an odour and/or a flavour or taste to a consumer product traditionally fragranced or flavoured, or of modifying the odour and/or taste of said consumer product.

By fragrance in this context is not only meant a fully formulated product fragrance, but also selected components of that fragrance, particularly those which are prone to loss, such as the so-called 'top notes'.

Top notes are defined by Poucher (Journal of the Society of Cosmetic Chemists 6(2):80 [1955]). Examples of well-known top-notes include citrus oils, linalool, linalyl acetate, lavender, dihydromyrcenol, rose oxide and cis-3-hexanol. Top notes typically comprise 15-25%wt of a fragrance composition and in those embodiments of the invention which contain an increased level of top-notes it is envisaged at that least 20%wt would be present within the particle.

Another group of fragrances with which the present invention can be applied are the so-called 'aromatherapy' materials. These include many components also used in fragrancery, including components of essential oils such as Clary Sage, Eucalyptus, Geranium, Lavender, Mace Extract, Neroli, Nutmeg, Spearmint, Sweet Violet Leaf and Valerian.

Typical fragrance components which it is advantageous to employ in the embodiments of the present invention include those with a relatively low boiling point, preferably those with a boiling point of less than 300, preferably 100-250 Celsius, measured at one atmosphere.

It is also advantageous to encapsulate fragrance components which have a low LogP (i.e. those which will be partitioned into water), preferably with a LogP of less than 3.0.

The pro-fragrance can, for example, be a food lipid. Food lipids typically contain structural units with pronounced hydrophobicity. The majority of lipids are derived from fatty acids. In these 'acyl' lipids the fatty acids are predominantly present as esters and include mono-, di-, triacyl glycerols, phospholipids, glycolipids, diol lipids, waxes, sterol esters and tocopherols.

The fragrance is typically present in an amount of from 10-85% by total weight of the particle, preferably from 15 to 75% by total weight of the particle. The fragrance suitably has a molecular weight of from 50 to 500 Dalton. Pro-fragrances can be of higher molecular weight, being typically 1-10 kD.

Pickering emulsion method:

"Pickering emulsion" as used herein refers to an emulsion that is stabilized by solid particles (for example colloidal silica) which adsorb onto the interface between the two phases. Pickering emulsion droplets are also suitable templates for micro-encapsulation and the formation of closed, non-permeable capsules.

This invention is also directed to a process to prepare the microcapsules.

Microcapsules in accordance with this invention are preferably prepared by using Pickering emulsion method.

Process for Preparation of microcapsules:

This invention is also directed to a process to prepare the microcapsules. The process comprises steps of:

a) Preparing an oil phase liquid comprising a silica precursor and a benefit agent;
b) Preparing an aqueous suspension by homogenizing the mixture of plate-like inorganic material and deionized water.
c) Preparing an o/w emulsion by adding the aqueous suspension of plate-like inorganic material into the oil phase liquid and homogenized to form emulsion.
d) Putting the o/w emulsion into 40°C oven for at least 24 h to get the microcapsule slurry product; wherein oil phase liquid of step a) comprises a thickening agent.

It is preferred that the silica precursor is selected from alkoxysilane and water-soluble silicate. Preferably, the alkoxysilane is selected from tetraethyl orthosilicate, tetramethyl orthosilicate, methyl triethoxysilane, methyl trimethoxysilane, vinyl trimethoxysilane, 3-aminopropyl trimethoxysilane, aminopropyl triethoxysilane or a mixture thereof. More preferably the alkoxysilane is selected from tetraethyl orthosilicate, aminopropyl triethoxysilane or a mixture thereof.

The oil phase liquid of step a) comprises a thickening agent which increases the viscosity of the oil phase liquid. The thickening agent can be a lipid which is solid at room temperature but is liquified via heat, or a mineral which can absorb oils and boost viscosity. Preferably, the thickening agent can be a lipid selected from fatty alcohol, fatty acid and wax. Preferably, the thickening agent can be a mineral selected from silica, bentonite and magnesium aluminum silicate. More preferably the thickening agent is hydrophobic silica.

It is preferred that ammonia is introduced into the aqueous suspension of step b).

Cosmetic composition

In accordance with a further aspect is disclosed a cosmetic composition comprising microcapsules of the first aspect in a cosmetically acceptable carrier. It is preferred that the cosmetic composition comprises from 0.001 % to 10%, more preferably from 0.005% to 7.55%, most preferably from 0.01% to 5% by weight microcapsule, based on total weight of the composition.

A variety of materials may be present in the compositions comprising the microcapsules of this invention to serve as cosmetically acceptable carriers.

Preferably the carrier comprises water. Amounts of water may, for example, range from 1 to 85 wt%, more preferably from 5 to 90%, even more preferably from 35 to 80%, optimally between 40 and 70% by weight of the cosmetic composition, depending on the nature of the composition. Preferably the carrier comprises a surfactant.

The cosmetic compositions of the invention may further comprise other ingredients which are common in the art to enhance physical properties and performances. Suitable ingredients include but are not limited to binders, colorants and pigments, pH adjusting agents, preservatives, optics, perfumes, viscosity modifiers, biological additives, buffering agents, conditioners, natural extracts, essential oils and benefit agents including anti-inflammatory agents, cooling agents, antiperspirant agents, anti-aging agents, anti-acne agents, anti-dandruff agents, hair conditioning agents, anti-microbial agents and antioxidants.

The cosmetic composition of this invention is a composition suitable for topical application to human skin, scalp/hair, including leave-on and wash-off products. It is preferred that the compositions of the invention are wash-off compositions. It is more preferred that the cosmetic composition of this invention is a hair care composition.

Hair care composition

Preferably, the hair care product is a shampoo, hair conditioner, hair cream, hair serum, mousse, hair gel or hair oil.

The hair care composition in accordance with this invention may also comprise antidandruff agent. The hair care compositions of the invention preferably comprise 0.05 to 5 wt% of antidandruff agent. Antidandruff agents are compounds that are active against dandruff and are typically antimicrobial agents and preferably antifungal agents. Antifungal agents typically display a minimum inhibitory concentration of about 50 mg/ml or less against *Malassezia spp.*

The antidandruff agent is preferably selected from azoles, Octopirox® (piroctone olamine), selenium sulfide, salicylic acid and combinations thereof. Azoles include ketoconazole and climbazole, preferably climbazole.

The hair care compositions of the invention may further comprise a zinc salt. The additional zinc salt may suitably be selected from zinc salts of organic acids, zinc salts of inorganic acids, zinc oxides, zinc hydroxides or a mixture thereof.

Examples of preferred zinc salts include zinc oxide, zinc pyrrolidone carboxylic acid, zinc citrate, zinc carbonate, zinc chloride, zinc sulphate, zinc glycinate, zinc acetate, zinc lactate, and mixtures thereof. When present, it is preferred that the hair care composition of the invention comprise 0.1 to 5 wt%, preferably 0.2 to 3 wt%, more preferably from 0.25 to 2.5 wt% of the salt based on the total weight of the composition.

The hair care composition of the present invention comprises a surfactant selected from the group consisting of anionic surfactants, nonionic surfactants, zwitterionic surfactants and mixtures thereof. The nature, type, amount and specific combinations that may be used depend on the formulation of the composition and would largely depend on whether it is a shampoo or a conditioner or a conditioning shampoo.

Preferably, the hair care composition of the invention is a shampoo. Preferably it comprises a surfactant which is sodium lauryl sulphate, sodium lauryl ether sulphate, sodium lauryl ether sulphosuccinate, ammonium lauryl sulphate, ammonium lauryl ether sulphate, sodium cocoyl isethionate and lauryl ether carboxylic acid, coco betaine, cocamidopropyl betaine, sodium cocoamphoacetate or a mixture thereof.

Preferably, the hair care composition of the present invention comprises from 1 to 50%, preferably from 2 to 40%, more preferably from 4 to 25% total surfactant.

It is further preferred that the hair care composition of the invention comprises a cosmetic ingredient. Preferably the cosmetic ingredient is selected from the group consisting of a silicone, an antibacterial agent other than antidandruff agents, a foam booster, a perfume, encapsulates (for example encapsulated fragrance) a dye, a colouring agent, a pigment, a preservative, a thickener, a protein, a phosphate ester, a buffering agent, a pH adjusting agent, a pearlescer (for example; mica, titanium dioxide, titanium dioxide coated mica, ethylene glycol distearate (INCI glycol distearate)) and/or opacifier, a viscosity modifier, an emollient, a sunscreen, an emulsifier, a sensate active (for example menthol and menthol derivatives), vitamins, mineral oils, essential oils, lipids, natural actives, glycerin, natural hair nutrients such as botanical extracts, fruit extracts, sugar derivatives and amino acids, microcrystalline cellulose and mixtures thereof.

Preferably, the hair care composition of the present invention includes from 0.01 to 20 wt% of the at least one cosmetic ingredient, more preferably from 0.05 to 10 wt%, still more preferably from 0.075 to 7.5 wt% and most preferably, from 0.1 to 5 wt% of the at least one cosmetic ingredient, by weight of the total composition.

The hair care composition of the present invention may also comprise synergistic antimicrobial compounds that give synergistic antimicrobial benefit when used in combination with the antidandruff active (for example zinc pyrithione) to enhance its properties and further inhibit the growth of Malassezia furfur. Non-limiting examples of these compounds include compounds having alcoholic groups (e.g. honokiol, magnolol or paeonol), Piperazines and a phenolic compound found in natural plant extract viz. thymol and terpeniol.

The haircare product may additionally comprise a vitamin B3 compound. The preferred vitamin B3 compound is niacinamide.

Niacinamide is known for secretion of AMPs (Anti-Microbial Proteins) from keratinocytes. The AMPs thus secreted provides for improving immunity of e.g. the scalp. Thus with the use of niacinamide, the anti-dandruff efficacy can be enhanced not just through anti-fungal activity but by boosting the scalp's own protection shield against germs, through use of niacinamide. This combination could provide further long-lasting protection e.g. up to 24 hours of protection against germs.

When present, it is preferred that the haircare compositions of the invention comprise 0.1 to 5% niacinamide, more preferably 0.5 to 5%, furthermore preferably 0.5 to 3%, and optimally 1.0 to 3.0% by weight of the composition.

Silicone

It is preferred that the hair care composition of the invention comprises silicone.

For example, the compositions of the invention may contain emulsified droplets of a silicone conditioning agent for enhancing conditioning performance.

Suitable silicones include polydiorganosiloxanes, polydimethylsiloxanes which have the CTFA designation dimethicone. In addition, suitable for use in the compositions of the invention (particularly shampoos and conditioners) are polydimethyl siloxanes having hydroxyl end groups, which have the CTFA designation dimethiconol.

Preferably the viscosity of the emulsified silicone is at least 10,000 cst at 25 °C, the viscosity of the silicone is preferably at least 60,000 cst, most preferably at least 500,000 cst, ideally at least 1,000,000 cst. Preferably the viscosity does not exceed $10^9$ cst for ease of formulation.

Examples of suitable pre-formed emulsions include Xiameter MEM 1785 and microemulsion DC2-1865 available from Dow Corning. These are emulsions /microemulsions of dimethiconol. Cross-linked silicone gums are also available in a pre-emulsified form, which is advantageous for ease of formulation. A further preferred class of silicones for inclusion in shampoos and conditioners are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

Specific examples of amino functional silicones suitable for use in the invention are the aminosilicone oils DC2-8220, DC2-8166 and DC2-8566 (all ex Dow Corning).

It is preferred that the total amount of silicone is 0.01 to 10 %wt, more preferably 0.1 to 5 wt% and most preferably 0.5 to 3 wt%.

Shampoos

When the haircare product of the invention is a shampoo, it is generally aqueous, i.e. they have water or an aqueous solution or a lyotropic liquid crystalline phase as their major component.

Suitably, the shampoo composition comprises 50 to 98%, preferably from 60 to 92% water.

Preferably the shampoo composition comprises one or more cationic polymers for conditioning the hair.

Suitable cationic polymers include homopolymers which are cationically substituted or may be formed from two or more types of monomers. The weight average ($M_w$) molecular weight of the polymers will generally be between 100000 and 3 Million Daltons. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus, when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general, secondary and tertiary amines, especially tertiary, are preferred.

Amine substituted vinyl monomers and amines can be polymerised in the amine form and then converted to ammonium by quaternization.

The cationic polymers can comprise mixtures of monomer units derived from amine- and/or quaternary ammonium-substituted monomer and/or compatible spacer monomers.

Suitable (non-limiting examples of) cationic polymers include:

- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms, (as described in U.S. Patent 4,009,256);
- cationic polyacrylamides (as described in WO95/22311).

Other cationic polymers that can be used include cationic polysaccharide polymers, such as cationic cellulose derivatives, cationic starch derivatives, and cationic guar gum derivatives.

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14 and JAGUAR C17.

Mixtures of any of the above cationic polymers may be used.

It is preferred that the hair care composition of the invention comprises 0.01 to 5%, preferably from 0.02 to 1%, more preferably from 0.05 to 0.8% cationic polymer.

The hair care composition of the invention may additionally comprise a cationic deposition polymer which is a cationic polygalactomannans having an average molecular weight ($M_w$) of from 1 million to 2.2 million g/mol and a cationic degree of substitution of from 0.13 to 0.3.

The polygalactomannans are polysaccharides composed principally of galactose and mannose units and are usually found in the endosperm material of seeds from leguminous plants such as guar, locust bean, honey locust, flame tree, and other members of the *Leguminosae* family. Polygalactomannans are composed of a backbone of 1→4-linked β-D-mannopyranosyl main chain units (also termed mannoside units or residues) with recurring 1→6-linked α-D-galactosyl side groups (also termed galactoside units or residues) branching from the number 6 carbon atom of a mannopyranose residue in the polymer backbone. The polygalactomannans of the different *Leguminosae* species differ from one another in the frequency of the occurrence of the galactoside side units branching from the polymannoside backbone. The mannoside and galactoside units are generically referred to herein as glycoside units or residues. The average ratio of mannoside to galactoside units in the polygalactomannan contained in guar gum (hereinafter termed "guar") is approximately 2:1.

Suitable cationic polygalactomannans include guar and hydroxyalkyl guar (for example hydroxyethyl guar or hydroxypropyl guar), that has been cationically modified by chemical reaction with one or more derivatizing agents.

In a typical composition the amount of cationic polygalactomannans will generally range from about 0.05 to 1%, preferably from 0.1 to 0.8%, more preferably 0.2 to 0.6% by weight of the composition.

The haircare product of the invention may additionally comprise an anionic polymeric rheology modifier such as a carboxylic acid polymer.

The term "carboxylic acid polymer" in the context of this invention generally denotes a homopolymer or copolymer obtained from the polymerization of ethylenically unsaturated monomers containing pendant carboxylic acid groups (hereinafter termed "carboxylic monomers").

Suitable carboxylic monomers generally have one or two carboxylic acid groups, one carbon to carbon double bond and contain a total of from 3 to about 10 carbon atoms, more preferably from 3 to about 5 carbon atoms.

Specific examples of suitable carboxylic monomers include α-β-unsaturated monocarboxylic acids such as acrylic acid, methacrylic acid and crotonic acid; and α-β-unsaturated dicarboxylic acids such as itaconic acid, fumaric acid, maleic acid and aconitic acid. Salts, esters or anhydrides of the α-β-unsaturated mono- or dicarboxylic acids described above may also be used. Examples include half esters of α-β-unsaturated dicarboxylic acids with $C_{1-4}$ alkanols, such as monomethyl fumarate; cyclic anhydrides of α-β-unsaturated dicarboxylic acids such as maleic anhydride, itaconic anhydride and citraconic anhydride; and esters of acrylic acid or methacrylic acid with $C_{1-30}$ alkanols, such as ethyl acrylate, butyl acrylate, 2-ethylhexyl acrylate, dodecyl acrylate, hexadecyl acrylate, and octadecyl acrylate.

Optionally, other ethylenically unsaturated monomers can be copolymerized into the carboxylic acid polymer backbone. Example of such other ethylenically unsaturated monomers include styrene, vinyl acetate, ethylene, butadiene, acrylonitrile and mixtures thereof. Carboxylic acid polymers may preferably have a molecular weight of at least 1 million Daltons.

Suitable examples include crosslinked copolymers polymerized from $C_{1-4}$ alkyl acrylate or methacrylate (e.g. ethyl acrylate) with one or more comonomers selected from acrylic acid, methacrylic acid and mixtures thereof. Such materials may generally be referred to under the INCI name of Acrylates Copolymer. Commercially available examples include Aculyn® 33 from Rohm and Haas.

Also suitable are crosslinked copolymers polymerized from $C_{10-30}$ alkyl esters of acrylic or methacrylic acid with one or more comonomers selected from acrylic acid, methacrylic acid and their respective $C_{1-4}$ alkyl esters. Such materials may generally be referred to under the INCI name of Acrylates/C10-30 Alkyl Acrylate Crosspolymer. Commercially available examples include Carbopol® polymers 1342 and 1382 from Lubrizol Advanced Materials.

Also suitable are optionally crosslinked copolymers of acrylic acid or methacrylic acid with alkyl acrylates and ethoxylated hydrophobically modified alkyl acrylates. Such materials may generally be referred to under the INCI names of Acrylates/Steareth-20 Methacrylate Copolymer, Acrylates/Beheneth-25 Methacrylate Copolymer, Acrylates/Steareth-20 Methacrylate Crosspolymer and Acrylates/Palmeth-25 Acrylates Copolymer. Commercially available examples include Aculyn® 22, 28 or 88 from Rohm & Haas and Synthalen® from 3V Sigma.

It is preferred that the carboxylic acid is a Carbomer, such as homopolymers of acrylic acid crosslinked with an allyl ether of pentaerythritol or an allyl ether of sucrose.

Mixtures of any of the aforementioned material may also be used.

Preferably hair care composition of the invention comprises 0.1 to 3.0%, more preferably 0.4 to 1.5% carboxylic acid polymer by weight of the composition.

In formulations containing anionic polymeric rheology modifiers such as the carboxylic acid polymers described above, it is often necessary to neutralize at least a portion of the free carboxyl groups by the addition of an inorganic or organic base. Examples of suitable inorganic or organic bases include alkali metal hydroxides (e.g. sodium or potassium hydroxide), sodium carbonate, ammonium hydroxide, methylamine, diethylamine, trimethylamine, monoethanolamine, triethanolamine and mixtures thereof.

The hair care composition of the invention may also comprise a nonionic polymeric rheology modifier which is selected from one or more nonionic cellulose ethers.

Suitable nonionic cellulose ethers or use as the nonionic polymeric rheology modifier in the invention include ($C_{1-3}$ alkyl) cellulose ethers, such as methyl cellulose and ethyl cellulose; hydroxy ($C_{1-3}$ alkyl) cellulose ethers, such as hydroxyethyl cellulose and hydroxypropyl cellulose; mixed hydroxy ($C_{1-3}$ alkyl) cellulose ethers, such as hydroxyethyl hydroxypropyl cellulose; and ($C_{1-3}$ alkyl) hydroxy ($C_{1-3}$ alkyl) cellulose ethers, such as hydroxyethyl methylcellulose and hydroxypropyl methylcellulose.

Preferred nonionic cellulose ethers for use as the nonionic polymeric rheology modifier in the invention are water-soluble nonionic cellulose ethers such as methylcellulose and hydroxypropyl methylcellulose. The term "water-soluble" in this context denotes a solubility in water of at least 1 grams, more preferably at least 3 grams, most preferably at least 5 grams in 100 grams of distilled water at 25° C. and 1 atmosphere. This level indicates production of a macroscopically isotropic or transparent, coloured or colourless solution.

Methyl cellulose and hydroxypropyl methylcellulose are commercially available in a number of viscosity grades from Dow Chemical as their METHOCEL® trademark series.

Mixtures of any nonionic cellulose ethers may also be suitable. In a typical composition according to the invention the level of nonionic cellulose ethers will generally range from about 0.01 to about 2.0%, and preferably ranges from 0.1 to 0.5%, more preferably from 0.1 to 0.3%, by weight based on the total weight of the composition.

Preferably the haircare product of the invention comprises 0.1 to 0.3% by weight nonionic cellulose ether.

The hair care composition of the invention may contain further optional ingredients to enhance performance and/or consumer acceptability. Examples of such ingredients

include fragrance, dyes and pigments and preservatives. Each of these ingredients will be present in an amount effective to accomplish its purpose. Generally, these optional ingredients are included individually at a level of up to 5% by weight based on the total weight of the composition.

Mode of Use

The cosmetic composition of the invention is primarily intended for topical application to skin, scalp and hair.

The present disclosure also provides a method of treating a substrate, the method comprising a step of treating the substrate with a composition comprising the microcapsule of the present invention. Preferably the substrate is skin, hair and/or scalp. More preferably, the substrate is hair and/or scalp.

The cosmetic composition is preferably hair care composition and when the hair care composition is a shampoo, it is topically applied to the hair and then massaged into the hair and scalp. Then it is rinsed with water prior to drying the hair. A hair oil or hair serum, being leave-on hair care compositions, are left on for 1 to 10 hours after application before being washed off.

The invention will be further illustrated by the following, non-limiting Examples, in which all percentages quoted are by weight based on total weight unless otherwise stated.

The invention is not limited to the embodiments illustrated in the drawings. Accordingly, it should be understood that where features mentioned in the claims are followed by reference numerals, such numerals are included solely for the purpose of enhancing the intelligibility of the claims and are in no way limiting to the scope of the claims. The examples are intended to illustrate the invention and are not intended to limit the invention to those examples *per se.*

## Examples

Materials:

| Material | Supplier |
|---|---|
| Limonene | Tokyo Chemical Industry Co., Ltd |
| Hydrophobic $SiO_2$ (AEROSIL R816) | Evonik Industries |
| Aminopropyl-triethoxysilane (APTES) | Aladdin Industrial Corporation |
| Tetraethyl orthosilicate (TEOS) | Shanghai Ling Feng Chemical Reagent Co., Ltd. |
| Ethanol | General Reagent |

Other chemicals used in the examples were obtained from Sinopharm Chemical Reagent Co., Ltd.

Example 1: Preparation of the microcapsule inside the invention.

(1) Preparation of plate-like MgAl-LDH material:
$Mg(NO_3)_2.6H_2O$ (0.0115 mol, 5.128 g), $Al(NO_3)_3.9H_2O$ (0.005 mol, 3.751 g) and hexamethylenetetramine (0.026 mol, 3.645 g) were mixed with 80 ml deionized water and the mixture was magnetically stirred until all chemicals dissolved completely. The solution was transferred into an autoclave and heated at 140 °C for 24 hours. Then, the mixture in autoclave was separated into 10 ml plastic centrifuge tube and crude product was gained through centrifugation. The crude product was then washed with ethanol solution (ethanol: deionized water=1:1 v/v) for 3 minutes under stirring speed 4k rpm, and the supernatant was discarded after centrifugation. The wash/centrifugation process was repeated for 3 times and finally the product was dried in a 50°C oven.

(2) Preparation of plate-like SDS (sodium dodecyl sulfonate) modified MgAl-LDH material:
The preparation process was similar as that of MgAl-LDH material (see above) except that sodium dodecyl sulfonate (0.0052 mol, 1.498 g) was also added in the mixing step.

(3) Preparation of microcapsules using MgAl-LDH material as stabilizer:

a) Preparation of oil phase liquid (A): 0.5 ml tetraethyl orthosilicate and 0.05 ml aminopropyltriethoxysilane were mixed with 2 ml limonene, and the mixture was shaken until completely homogeneous. In some cases, 0.034 g hydrophobic $SiO_2$ was added into the mixture, in order to increase the viscosity of the oil phase liquid.
b) Preparation of emulsifier solution (B): 0.4 g MgAl-LDH particle (or SDS modified MgAl-LDH particle) was added into 20 ml deionized water and the mixture was homogenized at 3k rpm for 30 seconds. In some cases, 0.25 ml ammonia was added into the mixture, in order to accelerate the reaction speed of shell fabrication.
c) Then, solution B was added into liquid A, and the mixture was homogenized at 8k rpm for 3 minutes to form emulsion.
d) Then the emulsion was put into 40°C oven for at least 24 hours to get the microcapsule slurry product.

(4) Characterization of prepared microcapsules:
The morphologies of the MgAl-LDH materials were characterized using a transmission electron microscopy (TEM, JEOL JEM-2011) at 200 kV. X-ray powder diffraction (XRD) analysis was conducted on a BRUKER D2-PHASER X-ray diffractometer at a scanning rate of 4/min over the $2\theta$ range of 10°- 70°, employing CuK$\alpha$ radiation (I=0.15418 nm). The morphologies of the microcapsule samples were characterized using scanning electron microscopy (SEM, PHENOM PROX), or observed using microscopy (Shanghai precision instrument co. LTD XSP-3C with 20× objective lens). Size of microcapsules was evaluated on Mastersizer 2000 Particle Size.

In order to further test the strength of the shell, perfume that in the microcapsule was removed by washing the microcapsule aqueous suspension by using ethanol. The strength of the microcapsule can be evaluated by observing whether the microcapsule structure collapsed after the ethanol treatment. If the shell of microcapsules could remain intact even after the ethanol treatment, the strength of shell is very good.

The microcapsules of the invention prepared by the above method were observed under microscope and a summary could be found in below Table 1

Table 1

| Microcapsule Reference No. | Platelike material | Thickness of platelike material | Core | Step b) of process | Morphology of microcapsule | Size ($\mu$m) |
|---|---|---|---|---|---|---|
| 1 | MgAl-LDH | ~500nm | limonene | - | with some dents on shell, slight phase separation | 5-30 |
| 2 | SDS modified MgAl-LDH | ~20nm | limonene | - | with some dents on shell | 5-20 |
| 3 | SDS modified MgAl-LDH | ~20nm | limonene + $SiO_2$ (thickener) | - | Intact microcapsule, well-dispersed | 5-30 |
| 4 | SDS modified MgAl-LDH | ~20nm | limonene +$SiO_2$ (thickener) | Include aqueous ammonia | Intact microcapsule, well-dispersed | 5-20 |

Microcapsule Reference No. 1-2 are not according to the invention. As can be seen from the Table 1, Microcapsule Reference No. 1-4 could be formed by using Pickering emulsion method. It also indicates that the thickness of platelike inorganic material could be reduced because of the anionic surface modification and it can help stabilizing the emulsion and thereby the formed microcapsule is more intact. It is also found that when the core comprises a thickener (e.g. silica) for oil phase liquid, microcapsules with better quality could be formed.

It is also found that the shell of microcapsule (Reference No.4) could remain intact even after the ethanol treatment, so the strength of shell of microcapsule (Reference No.4) is higher than the others. The thickness of shell was about 100-300nm.

Example 2: Perfume loading efficiency and loading rate of microcapsule of the invention (taking Reference No.4 as example).

A typical procedure was described as following: 0.5 ml microcapsule (Reference No.4) slurry was mixed with 2 ml deionized water under gentle stirring using a glass rod. Then the diluted slurry was filtered utilizing a syringe driven filter (0.45 $\mu$m, Polyethersulfone membrane, ANPEL scientific Instrument Co., Ltd.) to remove the microcapsules. The filtrate was mixed with 4 ml ethanol in a 20 ml vial. The amount of perfume in the filtrate (unloaded perfume) was evaluated using GC-FID and the data was recorded as $W_u$. In a separated case, 0.5 ml microcapsule slurry was mixed with 2 ml deionized water. Then 1.0 ml diluted slurry and 4.0 ml ethanol were added into a 20 ml vial. The vial was sealed and vibrated on IKA MS2 Minishaker at 1000 rpm for 1 min. The amount of perfume in the slurry (total perfume) was evaluated using GC-FID and the data was recorded as $W_T$. The perfume loading efficiency could then be calculated according to below equation

$$\% \text{ perfume loading efficiency} = (W_T-W_U)/ W_T *100 \text{ (Eq. 1)}$$

Where:

$W_T$= weight of total perfume
$W_U$=weight of unloaded perfume

In addition, the perfume loading rate could also be calculated according to below equation

$$\% \text{ perfume loading rate} = (W_T - W_U)/(W_s + W_T - W_U) *100 \text{ (Eq. 2)}$$

Where:

$W_s$= weight of capsule shell, which was calculated according to feed amount of the raw materials for shell formation.

The data is summarised in Table 2.

**Table 2**

| Microcapsule (Reference No.4) | Value |
|---|---|
| Weight of feeding amount of perfume (theoretical) | 840 mg |
| $W_T$ | 630 mg |
| $W_U$ | 0 mg (not detectable) |
| $W_S$ (theoretical) | 220 mg |
| Perfume loading efficiency | 100% |
| Perfume loading rate | 74% |

As shown in Table 2, the perfume loading efficiency is close to 100% since no unloaded perfume was detected. And the perfume loading rate was then calculated as 74%. Therefore, the perfume loading efficiency and loading rate of the microcapsule of the invention is quite good.

Example 3: Perfume leakage of microcapsule of the invention (taking Reference No.4 as example) in the formulation.

A simplified body wash formulation (no perfume) is shown in Table 3

**Table 3**

| Ingredients | wt% of the Ingredient |
|---|---|
| Sodium Laureth Sulfate | 12.86 |
| Ocamidopropyl Betaine | 5.67 |
| Cocamide Mea | 1.35 |
| Acrylates Copolymer | 6 |
| Additive and Preservative | 1.95 |
| Water | To 87 |

A typical procedure for perfume leakage evaluation was described as below: 40 mg microcapsule (Reference No.4) (400 mg microcapsule slurry, solid content about 10%, perfume loading rate 74%) was put into a 20 ml vial, to which 3 ml body wash formulation was added to get the final perfume concentration around 10 mg/ml. The vial was sealed and vibrated on IKA MS2 Minishaker at 1000 rpm for 1.5 minutes to make the formulation and capsule mix well. Afterward, the vial was stood under room temperature for 2, 4, or 24 hours. The mixture was then diluted around 10 folds by mixing with 36ml deionized water and filtered utilizing a syringe driven filter (0.45 $\mu$m, Polyethersulfone membrane, ANPEL scientific Instrument Co., Ltd.) to remove capsules. The purpose of dilution is to reduce viscosity of the formulation samples to facilitate filtration. Then 2 ml of above filtrate was put into a 20 ml vial and mixed with 8 ml ethanol (in order to make sure the perfume concentration in the scope of the predetermined calibration curve for GC-FID evaluation), and the perfume amount in the filtrate (perfume leakage) was evaluated using GC-FID.

The data is summarised in Table 4.

**Table 4**

| Storage time in body wash formulation (hours) | Leakage percentage (%) |
|---|---|
| 2 | 22±3 |
| 4 | 19±1 |

(continued)

| Storage time in body wash formulation (hours) | Leakage percentage (%) |
|---|---|
| 24 | $29\pm1$ |

The data in Table 4 indicates that the leakage percentage was between 20-30% during 24h storage, and no obvious increase of leakage was observed with increase of storage time. Thus, the leakage of the prepared perfume capsule is at an acceptable level.

Example 4: Perfume release of microcapsule of the invention (taking Reference No.4 as example).

A typical procedure for perfume release evaluation was described as below: The microcapsule sample (Reference No.4) was prepared by putting 100 $\mu$l microcapsule slurry into a 20 ml vial. The vial (without cap) was stood under room temperature (25°C) in fume hood for 0, 1, 2, 3, 4, 5 or 6 hours. The control sample was prepared by mixing 1.0 g limonene, 2.3 g Tween-40 and 10.0 g deionized water together. The content of limonene and Tween-40 in the control sample were 7.5% and 17.3%, respectively. 100 $\mu$l of the control sample was put into a 20 ml vial, and the vial (without cap) was stood under room temperature (25°C) in fume hood for 0, 1, 2, 3, 4, 5 or 6 hours. Then 5 ml ethanol was added into the vials containing capsule sample or control sample, and the vials were sealed. The vial was then vibrated on IKA MS2 Minishaker at 1000 rpm for 60 seconds. The perfume amount in ethanol (perfume that not released) was evaluated using GC-FID.

The data is summarised in Table 5.

**Table 5**

| Sample Reference No. | Release of perfume percentage (%) at different time point | | | | | | |
|---|---|---|---|---|---|---|---|
| | 0 hour | 1 hour | 2 hours | 3 hours | 4 hours | 5 hours | 6 hours |
| Control | 0 | $59\pm8$ | $80\pm18$ | $99\pm1$ | $100\pm0$ | $97\pm3$ | $100\pm0$ |
| Microcapsule (Reference No.4) | 0 | $10\pm1$ | $17\pm1$ | $24\pm1$ | $34\pm12$ | $29\pm17$ | $56\pm14$ |

The data in Table 5 indicates that almost 100% perfume was released from the control sample within 3 hours, while only around 60% perfume was released from the capsule sample at 6 hours. Therefore, it indicates the long-lasting release property of the microcapsule of the invention.

**Claims**

1. A microcapsule comprising:

   (i) a core comprising a benefit agent; and
   (ii) a shell comprising silica; wherein said shell comprises plate-like inorganic materials having an average thickness of 1-1000nm; wherein said benefit agent is fragrance, pro-fragrance, hair conditioning agent, anti-dandruff agent, moisturizers, emollients, dyes and/or pigments, colour care additives including dye fixing agents, or a mixture thereof; wherein the core of said microcapsule comprises a thickening agent.

2. A microcapsule as claimed in claim 1 wherein average particle size of said plate-like inorganic material is from 10 to 2000nm in diameter.

3. A microcapsule as claimed in claim 1 or 2 wherein said plate-like inorganic material is selected from MgAl-layered double hydroxide, hydroxyapatite, diatomite, magnesium hydroxide, calcium hydroxide, zeolite MCM-22, boron nitride.

4. A microcapsule as claimed in any of claims 1 to 3 wherein said plate-like inorganic material is MgAl-layered double hydroxide.

5. A microcapsule as claimed in any of claims 1 to 4 wherein said plate-like inorganic material is anionic surface modified.

6. A microcapsule as claimed in any of claims 1 to 5 wherein said benefit agent is selected from a fragrance, pro-fragrance, or a mixture thereof.

7. A microcapsule as claimed in any of claims 1 to 6 wherein said microcapsule has an average particle size of 0.5 to 100 microns.

8. A microcapsule as claimed in any of claims 1 to 7 wherein said microcapsule is in the form of an aqueous suspension.

9. A process to prepare a microcapsule as claimed in any of claims 1 to 8 comprising the steps of:

   a) Preparing an oil phase liquid comprising a silica precursor and a benefit agent;
   b) Preparing an aqueous suspension by homogenizing the mixture of plate-like inorganic material and deionized water;
   c) Preparing an o/w emulsion by adding the aqueous suspension of plate-like inorganic material into the oil phase liquid and homogenized to form emulsion;
   d) Putting the o/w emulsion into 40°C oven for at least 24 h to get the microcapsule slurry product; wherein oil phase liquid of step a) comprises a thickening agent.

10. A cosmetic composition comprising microcapsules as claimed in any of claims 1 to 8 in a cosmetically acceptable carrier.

11. A composition as claimed in claim 10 wherein said composition comprises a surfactant.

12. A composition as claimed in claim 10 or 11 wherein said composition is a wash-off product.

13. A composition as claimed in claim 10 to 12 wherein said composition is a hair care composition.


**Patentansprüche**

1. Mikrokapsel, bestehend aus:

   (i) einem Kern, der einen Wirkstoff umfasst; und
   (ii) einer Schale, die Siliciumdioxid umfasst, wobei die Schale plattenförmige anorganische Materialien mit einer durchschnittlichen Dicke von 1-1000 nm umfasst; wobei der Wirkstoff ein Duftstoff, ein Pro-Duftstoff, ein Haarkonditionierungsmittel, ein Antischuppenmittel, Feuchtigkeitsmittel, Weichmacher, Farbstoffe und/oder Pigmente, Farbpflegezusätze, einschließlich Farbstofffixiermittel, oder eine Mischung davon ist, wobei der Kern der Mikrokapsel ein Verdickungsmittel umfasst.

2. Mikrokapsel, wie im Anspruch 1 beansprucht, wobei die durchschnittliche Partikelgröße des plattenförmigen anorganischen Materials 10 bis 2000 nm im Durchmesser ist.

3. Mikrokapsel, wie im Anspruch 1 oder 2 beansprucht, wobei das plattenförmige anorganische Material unter MgAl-geschichtetem Doppelhydroxid, Hydroxyapatit, Diatomit, Magnesiumhydroxid, Calciumhydroxid, Zeolith MCM-22, Bornitrid ausgewählt ist.

4. Mikrokapsel, wie in einem der Ansprüche 1 bis 3 beansprucht, wobei das plattenförmige anorganische Material MgAl-geschichtetes Doppelhydroxid ist.

5. Mikrokapsel, wie in einem der Ansprüche 1 bis 4 beansprucht, wobei das plattenförmige anorganische Material anionisch oberflächenmodifiziert ist.

6. Mikrokapsel, wie in einem der Ansprüche 1 bis 5 beansprucht, wobei der Wirkstoff unter einem Duftstoff, einem Pro-Duftstoff oder einer Mischung davon ausgewählt ist.

7. Mikrokapsel, wie in einem der Ansprüche 1 bis 6 beansprucht, wobei die Mikrokapsel eine durchschnittliche Partikelgröße von 0,5 bis 100 Mikrometer aufweist.

8. Mikrokapsel, wie in einem der Ansprüche 1 bis 7 beansprucht, wobei die Mikrokapsel in Form einer wässrigen Suspension vorliegt.

9. Verfahren zur Herstellung einer Mikrokapsel, wie in einem der Ansprüche 1 bis 8 beansprucht, umfassend die Schritte:

a) Herstellen einer flüssigen Ölphase, umfassend einen Siliciumdioxid-Vorläufer und einen Wirkstoff;
b) Herstellen einer wässrigen Suspension durch Homogenisieren der Mischung aus plattenförmigem anorganischem Material und entionisiertem Wasser;
c) Herstellen einer O/W-Emulsion durch Zugeben der wässrigen Suspension des plattenförmigen anorganischen Materials in die flüssige Ölphase und Homogenisieren zur Bildung einer Emulsion;
d) Einlegen der O/W-Emulsion für mindestens 24 h in einen Ofen mit 40°C, um das Mikrokapsel-Aufschlämmungsprodukt zu erhalten, wobei die flüssige Ölphase des Schritts a) ein Verdickungsmittel umfasst.

10. Kosmetische Zusammensetzung, umfassend Mikrokapseln, wie in einem der Ansprüche 1 bis 8 beansprucht, in einem kosmetisch akzeptablen Träger.

11. Zusammensetzung, wie im Anspruch 10 beansprucht, wobei die Zusammensetzung ein Tensid umfasst.

12. Zusammensetzung, wie im Anspruch 10 oder 11 beansprucht, wobei die Zusammensetzung ein abwaschbares Produkt ist.

13. Zusammensetzung, wie im Anspruch 10 bis 12 beansprucht, wobei die Zusammensetzung eine Haarpflegezusammensetzung ist.

## Revendications

1. Microcapsule comprenant :

(i) un noyau comprenant un agent bénéfique ; et
(ii) une enveloppe comprenant de la silice ; dans laquelle ladite enveloppe comprend des matériaux inorganiques de type plaque ayant une épaisseur moyenne de 1 à 1000 nm ; dans laquelle ledit agent bénéfique est un parfum, un pro-parfum, un agent revitalisant capillaire, un agent antipelliculaire, des agents hydratants, des émollients, des colorants et/ou des pigments, des additifs de soin de la couleur comprenant des agents de fixation de colorant, ou un mélange de ceux-ci ; dans laquelle l'enveloppe de ladite microcapsule comprend un agent épaississant.

2. Microcapsule selon la revendication 1 dans laquelle la taille de particule moyenne dudit matériau inorganique de type plaque est de 10 à 2000 nm de diamètre.

3. Microcapsule selon la revendication 1 ou 2 dans laquelle ledit matériau inorganique de type plaque est choisi parmi un hydroxyde double couche de MgAl, l'hydroxyapatite, la diatomite, l'hydroxyde de magnésium, l'hydroxyde de calcium, la zéolite MCM-22 et le nitrure de bore.

4. Microcapsule selon l'une quelconque des revendications 1 à 3 dans laquelle ledit matériau inorganique de type plaque est un hydroxyde double couche de MgAl.

5. Microcapsule selon l'une quelconque des revendications 1 à 4 dans laquelle ledit matériau inorganique de type plaque est modifié en surface de manière anionique.

6. Microcapsule selon l'une quelconque des revendications 1 à 5 dans laquelle ledit agent bénéfique est choisi parmi un parfum, un pro-parfum ou un mélange de ceux-ci.

7. Microcapsule selon l'une quelconque des revendications 1 à 6 dans laquelle ladite microcapsule a une taille de particule moyenne de 0,5 à 100 microns.

8. Microcapsule selon l'une quelconque des revendications 1 à 7 dans laquelle ladite microcapsule est sous la forme d'une suspension aqueuse.

9. Procédé pour préparer une microcapsule selon l'une quelconque des revendications 1 à 8, comprenant les étapes consistant à :

a) préparer un liquide en phase huileuse comprenant un précurseur de silice et un agent bénéfique ;
b) préparer une suspension aqueuse en homogénéisant le mélange de matériau inorganique de type plaque et d'eau déminéralisée ;
c) préparer une émulsion h/e en ajoutant la suspension aqueuse de matériau inorganique de type plaque dans le liquide en phase huileuse et homogénéisée pour former une émulsion ;
d) mettre l'émulsion h/e dans un four à 40 °C pendant au moins 24 h pour obtenir le produit en suspension de microcapsule ; dans lequel le liquide en phase huileuse de l'étape a) comprend un agent épaississant.

10. Composition cosmétique comprenant des microcapsules selon l'une quelconque des revendications 1 à 8 dans un véhicule cosmétiquement acceptable.

11. Composition selon la revendication 10 dans laquelle ladite composition comprend un tensioactif.

12. Composition selon la revendication 10 ou 11 dans laquelle ladite composition est un produit rinçable.

13. Composition selon les revendications 10 à 12 dans laquelle ladite composition est une composition de soin capillaire.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016177156 A1
- US 4009256 A
- WO 9522311 A

**Non-patent literature cited in the description**

- Fenaroli's Handbook of Flavour Ingredients. CRC Press, 1975
- M. B. JACOBS. Synthetic Food Adjuncts. 1947
- S. ARCTANDER. Fragrance and Flavour Chemicals. Montclair, 1969
- POUCHER. *Journal of the Society of Cosmetic Chemists,* 1955, vol. 6 (2), 80
- CTFA Cosmetic Ingredient Directory